(19) Europäisches Patentamt
European Patent Office
Office européen des brevets

(11) Publication number: **0 147 980**
**A2**

(12) # EUROPEAN PATENT APPLICATION

(21) Application number: **84308652.1**

(22) Date of filing: **12.12.84**

(51) Int. Cl.[4]: **C 07 D 307/71**
A 61 K 31/34

(30) Priority: **13.12.83 US 561113**

(43) Date of publication of application:
**10.07.85 Bulletin 85/28**

(84) Designated Contracting States:
**AT BE CH DE FR GB IT LI LU NL SE**

(71) Applicant: **Rossignol, Jean Francois**
**8618 Germantown avenue**
**Philadelphia Pennsylvania 19118(US)**

(72) Inventor: **Rossignol, Jean Francois**
**8618 Germantown avenue**
**Philadelphia Pennsylvania 19118(US)**

(74) Representative: **Waters, David Martin, Dr. et al,**
**Smith Kline & French Laboratories Ltd. Patent**
**Department Mundells**
**Welwyn Garden City Hertfordshire AL7 1EY(GB)**

(54) **A 5-nitro-2-furanyl derivative and its use as an anti-parasitic agent.**

(57) N-(4-methoxy-2-methylphenyl)-3-(5-nitro-2-furanyl) propenamide of structure (I).

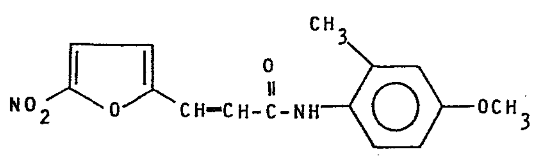

a process for its preparation, pharmaceutical compositions containing it and its use as an anti-parasitic agent are described.

EP 0 147 980 A2

0147980

A 5-nitro-2-furanyl derivative and its use as an anti-parasitic agent.

## BACKGROUND OF THE INVENTION

The present invention relates to the compound N-(4-methoxy-2-methyl phenyl)-3-(5-nitro-2-furanyl) propenamide having the structural formula (I)

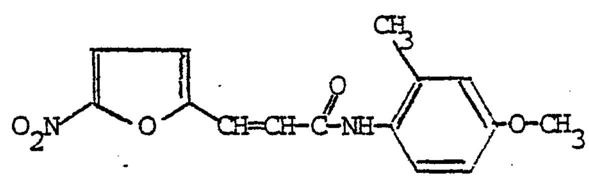

the use thereof to combat parasitic trematodes and anthelmintic compositions containing said compound.

U.S. 4,269,855 to Uhlendorf et al. describes ß-adrenolytic (3-alkylamino-2-hydroxy-propoxy)-furan-2-carboxylic acid anilides of the formula:

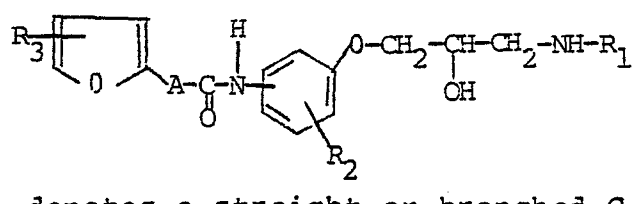

in which $R_1$ denotes a straight or branched $C_1$ to $C_5$ alkyl group or a cyclopropyl or cyclopentyl group, $R_2$ denotes a hydrogen or halogen atom or a methyl, methoxy, nitro or acetyl group, $R_3$ denotes a hydrogen or halogen atom or a nitro group and A denotes a single bond

or the $-CH_2-$ or $-CH=CH-$ group, and physiologically tolerated acid addition salts thereof which possess ß-adrenolytic properties and a low toxicity.

U.S. 3,982,007 describes a pharmaceutical antimicrobial composition including a benzisothiazolinone derivative and a 2-nitrofuryl derivative of the formula

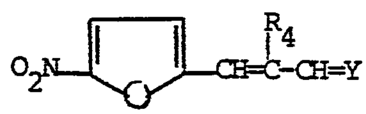

in which $R_4$, inter alia, could be $-CONHR_7$ where $R_7$ could, inter alia, be substituted or unsubstituted phenyl and Y is O or $-N-NH-\overset{Z}{\overset{\|}{C}}-NH_2$ with Z being S or =NH.

## SUMMARY OF THE INVENTION

Therefore, an object of the present invention is to provide a novel nitrofuran derivative characterized by antiparasitic activity.

Another object of the present invention is to provide a novel nitrofuran derivative suitable for use in the treatment of Schistosomiasis in humans.

Still another object of this invention is to provide pharmaceutically acceptable antiparasitic, particularly for killing infective worms of the genus Schistosoma, compositions and methods for their use.

Other objects of the invention will be apparent to the skilled artisan from the Detailed Description of the Invention, hereinbelow.

## DETAILED DESCRIPTION OF THE INVENTION

It has now been found that the novel compound N-(4-methoxy-2-methyl phenyl)-3-(5-nitro-2-furanyl) propenamide, having the structural formula (I)

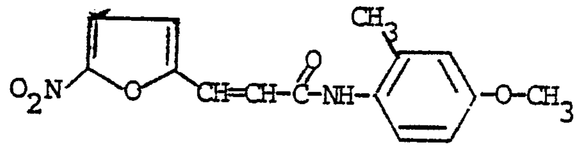

possesses parasiticidal activity, particularly against certain types of infective worms which heretofore have been difficult to eradicate in an efficient, acceptable manner. The compound of the formula (I) is suitable for use in treating Schistosomiasis in humans, for example, as caused by Schistosoma mansoni, Schistosoma hematoeum, Schistosoma intercalatum or Schistosoma japonicum. In addition, the compound of this invention can be used to treat Schistosoma species harbored by other mammals, such as various domestic and wild animal species.

The novel propenamide of this invention can be prepared in accordance with the following reaction scheme:

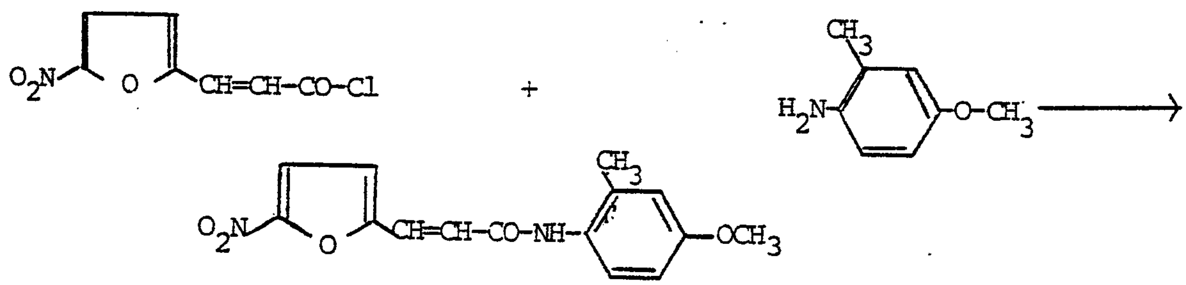

Nitrofuranyl acrylic acid, available from Apin Chemical, Oxon, United Kingdom, is converted to the acid chloride, which in turn is condensed with 4-methoxy-2-methylaniline in an appropriate organic solvent.

The product is recovered from chloroform in the form of a powder, which can be recrystallized from a solvent such as ethanol. Recrystallization from ethanol or methanol provides fine yellow-brown microcrystalline needles melting at approximately 150°C by decomposition.

## SYNTHESIS EXAMPLE

Fifteen grams of (5-nitro-2-furanyl) acrylic acid in 60 ml of chloroform was admixed with two molecular equivalents of thionyl chloride. The temperature of chlorination (an endothermic reaction) was maintained at about 22°C by slight heating. The acid chloride (16.8 grams), in chloroform, was obtained and was directly condensed through admixture with 11.23 g 4-methoxy-2-methyl -aniline (in 40 ml of chloroform), in the presence of a few drops of triethylamine at a temperature below 20°C.

Purification is carried out using conventional procedures. The reaction medium was heated to completely dissolve the reaction product. Thereafter, a two-stage washing was carried out, first with acidified water to remove traces of amine chlorides and then with tap water. Thereafter, the chloroform was evaporated under reduced pressure to yield 16.2 grams of compound (I). Recrystallisation from ethanol or methanol gave crystalline needles of N-(4-methoxy-2-methylphenyl)-3-(5-nitro-2-furanyl) propenamide, mp 150°C (decomp).

A suitable dosage range for the compound of this invention to combat Schistosomiasis is 25 to 50 mg/kg, one to three times a day, usually for one to ten days.

The invention includes within its scope pharmaceutical compositions comprising the compound of formula (I) in association with a customary pharmaceutically acceptable carrier. Such compositions may be, for example, in the form of tablets, dragees, solutions, emulsions, powders, capsules, or other forms. The compound of the invention can be administered orally or parenterally. Tablets may be obtained, for example, by mixing the active compound with known auxiliaries which are compatible with the compound of the formula (I), for example inert diluents, such as calcium carbonate, calcium phosphate or lactose, disintegrants, such as maize starch or alginic acid, binders such as starch or gelatin, lubricants, such as magnesium searate or talc, and agents for achieving a sustained release effect, etc.

Injectables can be formed using conventional fluid carriers, such as peanut oil, carboxymethylcellulose or water containing a suitable dispersant such as 1% Tween 80.

The following examples illustrate the pharmacological activities of the compound of this invention.

Example 1

Primary Prophylactic Test against Schistosoma mansoni in Mice

Introduction

The primary screening method used to evaluate prophylactic activity against Schistosoma mansoni is a mortality test. Mice are exposed to a massive number of cercariae by tail immersion. In untreated control mice this exposure produces a reproducible mortality pattern with 99+% of the mice dying between 20 and 30 days (mean survival time 24 days). The test compound is administered in a single dose two days after cercarial exposure. A compound is considered active if mice survive to 49 days, at which time they are sacrificed and examined for worms.

The test is of the "prophylactic"-type in the sense that the drug action is on the immature migrating schistosomes and prevents the development of adult worms in the portal circulation.

Methods

Mice weighing 18 to 23 grams were exposed to 3500-4500 infective S. mansoni cercariae by tail immersion for 30 minutes, and subsequently housed in groups of five per cage (5 test mice and 5 control mice). Two days after infection, the test compound of the formula (I) was administered subcutaneously as a single 1,280 mg/kg dose suspended in peanut oil to one group of five mice.

Mouse mortality was recorded daily for 49 days after infection. On the 49th day, survivors were sacrificed and the total worm burden was determined by perfusion of the liver and portal circulation (perf-o-suction technique).

Results

Control mice exposed to at least 3500 cercariae and not treated with the test compound (I) experience 99+% mortality, with nearly all deaths occurring between about day 20 and day 30. In treated mice, deaths occurring within one week after drug administration are assumed to be induced by the toxic action of the drug itself, since, control mice rarely die during this period. The survival of drug treated mice to 49 days is interpreted as evidence of drug action which kills or otherwise prevents the development of the migrating immature schistosomes. The survival of only one mouse has, in our experience, been difficult to reproduce, and, in general, a compound is not considered active unless two or more mice survive to 49 days.

In our experiment, two test mice died on day 41, i.e., about 21 to 11 days after the mortality time in control mice. Three mice survived until day 49 and were subsequently sacrificed.The worm burden was tabulated as indicated below:

| | Male | Female | Total |
|---|---|---|---|
| Mouse 1 | 10 | 5 | 15 |
| Mouse 2 | 12 | 7 | 19 |
| Mouse 3 | 10 | 6 | 16 |
| mean | 10.6 | 6.0 | 16.6 |

The compound is therefore considered active against the migrating stage of Schistosoma mansoni in mice following a single subcutaneous administration of 1,280

mg/kg of the test chemical. The control mice died at days 19, 22, 25, 28 and 32.

Example 2

Primary Curative Test against Schistosoma mansoni in Hamster

Introduction

The primary curative test evaluates the therapeutic activity of the drug against *S. mansoni* in hamster. Interpretation of the test is based upon observation of a shift of worms from the portal and mesenteric veins to the liver under the influence of the test drug.

Methods

Hamsters weighing 150 to 250 grams were exposed to 150-250 *S. mansoni* cercariae by the ring method. 30 to 35 days after infection, the test drug (compound (I)) was administered as a 200 mg/kg daily dose for four consecutive days. The drug was administered by oral gavage suspended in gum syrup. Hamsters were sacrificed three days after the last drug treatment. The liver was removed, squashed between two slides, and the number of live and dead worms in the liver were recorded.

Results

From experience, the total worm burden in the portal circulation of *S. mansoni* infected hamsters ranges from 20 to 50 worms, i.e., approximately 60% of the total number of cercariae injected. Over 80% of these worms reside in the extra-hepatic portal and mesenteric veins. Thus, when the liver is removed in control animals about 10 worms could be counted in the liver.

In our experiment including five treated hamsters and three controls, results are summarized as indicated below:

| Hamster No. | No. of worms found in the liver | | | |
|---|---|---|---|---|
| | Treated Animals | | Control Animals | |
| | M | F | M | F |
| 1 | 17 | 17 | | |
| 2 | 10 | 6 | | |
| 3 | 10 | 18 | | |
| 4 | 50 | 60 | | |
| 5 | 46 | 54 | | |
| 6 | | | 6 | 5 |
| 7 | | | 3 | 1 |
| 8 | | | 37 | 30 |
| Mean No. of worms | 26.6 | 31.0 | 15.3 | 12.0 |

The test drug produced a marked shift of the worms from the portal and mesenteric veins to the liver, where the parasites were dead. The effective dose is 200 mg/kg per day for four consecutive days.

Example 3

Primary Curative Test against S. japonicum in Hamsters

The primary curative test evaluates the therapeutic activity of the drug against *S. japonicum* in hamsters. Interpretation of the test is based upon observation of a shift of worms from the portal and mesenteric veins to the liver under the influence of the test drug. In addition the mortality of the worms found in the liver, portal and mesenteric veins is also an important criteria of effectiveness in establishing the schistosomicidal effectiveness of the drug.

Methods

Hamsters weighing 150 to 250 grams were exposed to 100 *S. japonicum* cercariae by the ring method. 35 to 45 days after infection, the test compound (I) was administered as a single 1 g dose by oral gavage, the product being suspended in gum syrup. Out of 10 infected animals,, 5 were so treated, with 5 being used as control animals.

Hamsters were sacrificed three days after treatment; the liver was removed as well as the portal and mesenteric veins, squashed between two slides and the number of live or dead worms in the liver, the portal and mesenteric veins was recorded.

Results

The total worm burden in the mesenteric and portal circulation of S. japonicum in the controls ranged from 25 to 40 worms, i.e., approximately up to 40% of the total number of cercariae administered. All parasites are located in the extra hepatic portal and mesenteric veins as illustrated in our control group. In our experiment including five treated and five control animals, results are summarized as indicated below:

| Animal No. | Type of Treatment | No. of worms found Liver Male | Liver Female | Mesenteric Male | Mesenteric Female | Portal Male | Portal Female | % dead |
|---|---|---|---|---|---|---|---|---|
| 1 | 1 g/kg | 8 | 7 | 7 | 5 | 0 | 0 | 100 |
| 2 | 1 g/kg | 14 | 6 | 6 | 4 | 0 | 0 | 100 |
| 3 | 1 g/kg | 8 | 4 | 4 | 2 | 0 | 0 | 70 |
| 4 | 1 g/kg | 14 | 6 | 8 | 4 | 0 | 0 | 100 |
| 5 | 1 g/kg | 9 | 6 | 7 | 4 | 0 | 0 | 60 |
| 6 | control | 0 | 0 | 6 | 3 | 5 | 2 | 0 |
| 7 | control | 0 | 0 | 9 | 4 | 14 | 7 | 0 |
| 8 | control | 0 | 0 | 11 | 6 | 11 | 8 | 0 |
| 9 | control | 0 | 0 | 7 | 5 | 7 | 6 | 0 |
| 10 | control | 0 | 0 | 10 | 8 | 15 | 7 | 0 |

These results suggest that the test drug is not only producing a marked shift of the worms from the portal and mesenteric veins to the liver but also having a schistosomicidal effect in killing on the average about 86% of the worms in the liver, the portal and mesenteric veins.

Acute Oral Toxicity Study in Mice

The test chemical was administered as a single 4,000 mg/kg oral dose to five male and five female Swiss mice. Based on clinical observation, daily performed for

14 days following treatment, weights recorded before, 7 and 14 days after treatment and macroscopic examination of the organs from the animals surviving until day 14, no adverse pattern was recorded.

The $LD_{50}$ of the test drug, when administered orally in mice, is greater than 4 g/kg.

Variations of the invention will be apparent to the skilled artisan.

Claims for BE, CH, DE, FR, GB, IT, LI, LU, NL AND SE

1. The compound of structure (I), namely, N-(4-methoxy-2-methylphenyl)-3-(5-nitro-2-furanyl) propenamide.

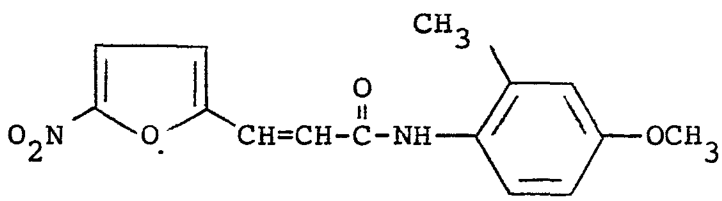

(I)

2. A pharmaceutical composition comprising the compound of claim 1 in association with a pharmaceutically acceptable carrier.

3. A pharmaceutical composition as claimed in claim 2 in a form suitable for oral administration.

4. A pharmaceutical composition as claimed in claim 2 in a form suitable for parenteral administration.

5. A process for the preparation of the compound of claim 1 which comprises reacting 5-nitro-2-furanyl acrylic acid chloride with 4-methoxy-2-methyl aniline.

6. N-(4-methoxy-2-methylphenyl)-3-(5-nitro-2-furanyl)propenamide for use as a medicament.

7. N-(4-methoxy-2-methylphenyl)-3-(5-nitro-2-furanyl)propenamide for use as an anti-parasitic agent.

8. N-(4-methoxy-2-methylphenyl)-3-(5-nitro-2-furanyl)propenamide for use in the treatment of Schistosomiasis.

9. The commercial use of N-(4-methoxy-2-methylphenyl)-3-(5-nitro-2-furanyl)propenamide as a therapeutic agent for the treatment of parasitic diseases.

Claims for AT

1. A process for the preparation of N-(4-methoxy-2-methylphenyl)-3-(5-nitro-2-furanyl) propenamide of the forumula (I):

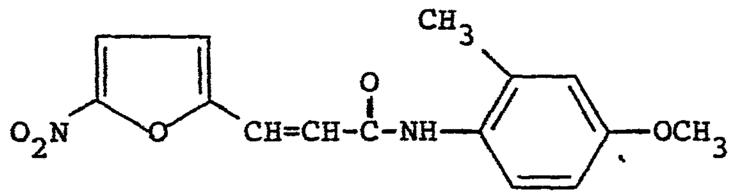

(I)

which comprises reacting (5-nitro-2-furanyl) acrylic acid chloride with 4-methoxy-2-methyl aniline.

2. The process of claim 1 in which the reaction is carried out in chloroform solution in the presence of triethylamine and at a temperature below 20°C.

3. A process for preparing a pharmaceutical composition which comprises bringing into association N-(4-methoxy-2-methylphenyl)-3-(5-nitro-2-furanyl) propenamide and a pharmaceutically acceptable carrier.